# EUROPÄISCHE PATENTSCHRIFT

(11) **EP 1 589 928 B1**
(45) Veröffentlichungstag und Bekanntmachung des Hinweises auf die Patenterteilung: **29.11.2006**
(21) Anmeldenummer: 04707920.7
(22) Anmeldetag: 04.02.2004
(51) Int. Cl.: A61J 7/00, G01N 3/04, G01N 3/20

(54) **VERFAHREN UND VORRICHTUNG ZUR PRÜFUNG DER TEILBARKEIT UND DER TEILGENAUIGKEIT VON TABLETTEN**
METHOD AND DEVICE FOR TESTING THE BREAKABILITY AND THE BREAKING ACCURACY OF TABLETS
PROCEDE ET DISPOSITIF POUR VERIFIER LA DIVISIBILITE ET LA PRECISION DE DIVISION DE COMPRIMES

(30) Priorität: 05.02.2003 DE 10304688
(43) Veröffentlichungstag der Anmeldung: 02.11.2005
(73) Patentinhaber: MERCKLE GMBH, 89143 Blaubeuren (DE)
(72) Erfinder: STREIL, Frank, 89143 Blaubeuren (DE)
(74) Vertreter: Best, Michael
(86) Internationale Anmeldenummer: PCT/EP2004/001033
(87) Internationale Veröffentlichungsnummer: WO 2004/069131

(56) Entgegenhaltungen:
- WO-A-01/90723
- DE-A- 2 503 683
- DE-U- 1 843 034
- DE-U- 9 109 852

## Beschreibung

Die Erfindung betrifft ein Verfahren, mit dem die Teilbarkeit und die Teilgenauigkeit von Tabletten reproduzierbar ermittelt werden kann, sowie eine Vorrichtung zur Durchführung dieses Verfahrens.

Bei der Dosierung von Medikamenten in Tablettenform soll häufig nicht eine ganze Tablette sondern nur ein Teil einer Tablette verabreicht werden. Hierdurch erhält der Arzt mehr Flexibilität in der Dosierungsmenge, und für einen Patienten ist folglich eine Kostenersparnis möglich, da es zwischen höher und niedrig dosierten Tabletten häufig kaum Preisunterschiede gibt.

Um dem Patienten bzw. dem Arzt das Teilen der Tabletten zu erleichtern, werden Tabletten hergestellt, die bereits zur Teilung vorgesehen sind. Solche Tabletten sind in der Regel mit einem mittig verlaufenden Einschnitt als Sollbruchstelle versehen. Es können auch mehrere solcher Sollbruchstellen auf einer Tablette vorhanden sein. Limitiert wird die Anzahl dieser Sollbruchstellen im Prinzip nur durch die Größe der Tablette. In der Regel ist allerdings nur eine Sollbruchstelle vorhanden. Die Sollbruchstelle wird im nachfolgenden Bruchkerbe genannt. Das Anbringen solcher Bruchkerben soll eine leichtere und gleichförmige Teilung der Tablette ermöglichen.

In der Praxis werden die Mehrzahl aller Tabletten vom Patienten mit der Hand geteilt. Teilbare Tabletten müssen also eine gute Teilbarkeit (d.h. an der Sollbruchstelle eine geringe Festigkeit) aufweisen, damit auch ältere oder geschwächte Patienten die Tabletten ohne größeren Aufwand teilen können.

Auch an die Teilgenauigkeit von Tabletten werden besondere Anforderungen gestellt, da die exakte Dosierung des Wirkstoffs von der Teilgenauigkeit der Tablette abhängt, und je nach Teiltechnik werden unterschiedliche Ergebnisse bei der Bestimmung der Gehalts- und Massengleichförmigkeit von teilbaren Tabletten erhalten. Der gleiche Effekt tritt auch auf, wenn verschiedene Personen die Tablette teilen (G. Kristensen, (Juni 1995) Pharmeuropa, Bd. 7, Nr. 2). Tabletten, die eine Bruchkerbe aufweisen, weisen zwar eine höhere Teilgenauigkeit auf, als Tabletten ohne eine derartige Bruchkerbe, auch bei der Teilung von Tabletten mit Bruchkerbe kann es aber zu unterschiedlich großen Teilen und damit zu einer Ungenauigkeit bei der Dosierung kommen. Dieses Problem ist seit langem bekannt und seit Mitte April 2002 ist es für Tabletten mit vorgesehener Teilung vorgeschrieben, daß nachgewiesen werden muß, daß die Teilstücke nach der Teilung den Anforderungen auf Gehaltsgleichförmigkeit bzw. Massengleichförmigkeit gemäß Europäischem Arzneibuch (Pharm.-Eur., Abschnitte 2.9.5 und 2.9.6) entsprechen. Das Europäische Arzneibuch gibt aber kein Verfahren an, mit dem bestimmt werden kann, daß die Teilstücke die erforderte Gehaltsgleichförmigkeit und Massengleichförmigkeit aufweisen.

Zum Teilen von Tabletten sind bereits mehrere Apparate und Methoden im Stand der Technik bekannt, welche die Tabletten mit einer Klinge zerteilen, oder welche das Zerteilen bzw. das Brechen der Tabletten von Hand erleichtern.

Die deutschen Patente DE 42 38 843 A1 und DE 199 57 015 A1 offenbaren Vorrichtungen zum Teilen von Tabletten, bei denen eine Tablette in einer Aussparung durch Schaumstoff oder durch Einklemmen zwischen die Seitenwände der Aussparung fixiert und dann durch eine Klinge geteilt wird, die auf die Tablette gedrückt wird.

Das deutsche Patent DE 196 41 495 offenbart einen Tablettenteiler, der im wesentlichen aus einem wannenförmigen Gehäuseteil, einem stiftförmigen Druckkörper und einem an dem wannenförmigen Gehäuseteil angelenkten Deckelteil besteht. Das Deckelteil preßt beim Verschließen den stiftförmigen Druckkörper und eine im Gehäuseteil eingelegte Tablette so gegeneinander, daß hierdurch die Tablette zerspringt.

Das US-Patent 4,422,553 offenbart einen Tablettenbehälter, der zum Teilen von Tabletten verwendet werden kann. Der Behälter besitzt einen Bauteil, integriert oder abnehmbar, an dem eine Schneidekante angebracht ist. Die Schneidekante wird auf die Tablette gedrückt, wodurch diese am Druckpunkt zerbricht.

Das deutsche Patent DE 199 46 088 A1 offenbart eine Vorrichtung zum Teilen von Tabletten die das Teilen von Hand erleichtern und unterstützen soll. Die Tabletten werden in Mulden gegeben welche so geformt sind, daß sie eine halbe auf dem Rande stehende Tablette aufnehmen können. Die freie, überstehende Hälfte der Tablette kann dann von Hand gebrochen werden.

Das US-Patent 4,330,936 offenbart eine Vorrichtung zum sicheren Halten einer Tablette, so daß diese leicht von Hand in gleiche Teile zerteilt werden kann. Die Vorrichtung besteht im wesentlichen aus pinzettenartigen Klemmbacken, an deren Ende sich eine Auflagefläche befindet. Zum Zerteilen der Tablette wird diese so auf die Auflagefläche gelegt, daß der abzuteilende Teil nach außen ragt. Durch Zusammendrücken der Klammern wird die Tablette fixiert und das herausragende Teil der Tablette kann von Hand abgebrochen werden.

Das US-Patent 5,417,359 offenbart eine Vorrichtung zum Teilen von Tabletten, die in Form einer Karte ausgestaltet ist. Diese Karte ist mit einer oder mehreren Erhebungen versehen, die das Brechen einer Tablette unterstützen.

Alle im Stand der Technik beschriebenen Geräte und Vorrichtungen sind darauf ausgelegt dem Patienten das Teilen von Tabletten zu erleichtern. Bei der Entwicklung der meisten Geräte wurde darauf geachtet, daß sie praktisch sind, d.h. klein und leicht bedienbar. Keine der Vorrichtungen ist darauf ausgelegt, reproduzierbar die Teilbarkeit und Teilgenauigkeit von Tabletten zu bestimmen, und ein Verfahren zur Bestimmung der Teilbarkeit und der Teilgenauigkeit von Tabletten wird auch in keiner der genannten Druckschriften vorgeschlagen.

Allen diesen Vorrichtungen ist gemein, daß sie letztlich von Hand betätigt werden und daher wie beim manuellen Zerbrechen der Tabletten die Teilgenauigkeit letztlich vom Geschick der das Gerät bedienenden Person abhängt.

Die WO 01/90723, gegenüber welcher die Ansprüche 1 und 6 abgegrenzt sind, offenbart eine Vorrichtung zur Prüfung der Bruchfestigkeit und/oder der Bruchgenauigkeit von Spalttabletten. Die Vorrichtung weist zwei gegenüberliegende zueinander bewegliche Preßbacken auf, die jeweils an ihren der gegenüberliegenden Preßbacke zugewandten Stirnseiten zumindest einen Vorsprung aufweisen, die gegeneinander versetzt angeordnet sind. Eine Vorrichtung mit einem Fixierstift, der keine Kraft ausübt, ist in der Druckschrift nicht offenbart.

Vorrichtungen zum Ermitteln der Druckfestigkeit und anderer Parameter von Tabletten sind beispielsweise auch aus der US-A 4,409,843, der DE 297 11 490 U1, der DE-A 33 15 397 und der DE-A 25 03 683 bekannt.

Es besteht ein Bedarf nach einem Verfahren, mit dem man einerseits feststellen kann, ob eine Tablette überhaupt teilbar ist (d.h. von einem Patienten ohne größeren Aufwand in zwei Teile zerbrochen werden kann), aber auch ob die Tablette gleichförmig geteilt werden kann, so daß die Anforderungen des Europäischen Arzneibuches bezüglich der Gehaltsgleichförmigkeit und der Massengleichförmigkeit erfüllt werden. Das Verfahren sollte die in der Praxis auftretenden Bruchbedingungen von Tabletten möglichst genau simulieren und dabei reproduzierbare und von dem Bedienpersonal unabhängige Ergebnisse liefern. Ferner sollte das Verfahren auch Informationen über die Kraft liefern, die ein Patient aufwenden muß, um eine Tablette an der vorgesehenen Sollbruchstelle zu teilen. Ferner besteht Bedarf nach einer Vorrichtung, mit der ein derartiges Verfahren vorteilhafterweise ausgeführt werden kann.

Aufgabe der Erfindung ist es daher, ein Verfahren zur Verfügung zu stellen, mit dem die Teilbarkeit und die Teilgenauigkeit von Tabletten bestimmt werden kann, sowie eine Vorrichtung zur Durchführung des Verfahrens.

Diese Aufgabe wird erfindungsgemäß durch den Gegenstand der Patentansprüche gelöst.

Erfindungsgemäß wird daher ein Verfahren zur Bestimmung der Teilbarkeit und der Teilgenauigkeit einer Tablette zur Verfügung gestellt, bei dem eine Tablette, die auf zumindest einer Seite zumindest eine Bruchkerbe aufweist, entlang dieser Bruchkerbe gebrochen wird. Das Verfahren ist dadurch gekennzeichnet, daß an der Seite der Tablette, die diese Bruchkerbe aufweist, ein Fixierstift entlang der Bruchkerbe angelegt wird, so daß die Tablette mit der Seite, die der Seite gegenüberliegt, die diese Bruchkerbe aufweist, gegen eine Bruchkante fixiert wird. Die Fixierung erfolgt derart, daß die Bruchkante und der Fixierstift parallel zueinander liegen. Anschließend wird über Bruchbacken, die beidseitig des Fixierstifts auf die Tablette einwirken, eine Kraft an die Tablette angelegt, so daß die Tablette entlang der Bruchkerbe bricht.

Aus der bis zum Bruch erforderlichen Kraft kann dann ohne weiteres geschlossen werden, wie gut eine Tablette teilbar ist, und ob beispielsweise ein bestimmter Grenzwert eingehalten wird. Durch geeignete Untersuchungen, z.B. Bestimmung des Gewichts der Tablettenhälften oder der Menge an Bestandteilen in den einzelnen Tablettenhälften oder optische Untersuchungen der Tablettenhälften kann dann bestimmt werden, wie hoch die Teilgenauigkeit der Tablette ist, das heißt wie stark die beiden Tablettenhälften in ihrem Gewicht, ihrer Größe, ihrer chemischen Zusammensetzung, etc. voneinander abweichen.

Die Kraft wirkt von der Seite auf die Tablette ein, an der der Fixierstift anliegt, d.h. auf der Seite, auf der sich der Fixierstift befindet. Die Tablette wird damit gegen die Bruchkante gedrückt, bis sie entlang der Bruchkerbe bricht.

Die Übertragung der Kraft erfolgt über Bruchbacken, die beidseitig des Fixierstifts an die Tablette angreifen und die auf geeignete Art und Weise, beispielsweise über eine Vorschubeinrichtung, die vorteilhafterweise mit einem Schrittmotor gekoppelt ist, bewegt werden. Die für das Brechen der Tablette erforderliche Kraft wird daher nicht über den Fixierstift übertragen, der nur zur Fixierung der Tablette dient. Indem man die Vorschubeinheit mit einem Dehnungsmeßstreifen ausstattet, mit dem beim Bruchvorgang die Brechkraft der Tablette bestimmt werden kann, ist es auch auf einfache Art und Weise möglich, die Bruchfestigkeit der Tablette zu bestimmen. Bevorzugt ist der Dehnungsmeßstreifen mit dem Schrittmotor so gekoppelt, daß beim Brechen der Tablette und dem damit verbundenen Absinken der Druckkraft die Bruchbacken in Gegenrichtung bewegt werden. Die Bruchfestigkeit der Tablette kann natürlich auch durch Kombination anderer üblicher Meßverfahren, die dem Durchschnittsfachmann bekannt sind, mit dem erfindungsgemäßen Verfahren bestimmt werden.

Die Mittel, mit denen der Fixierstift auf die Bruchkerbe der Tablette einwirkt und diese an die Bruchkante fixiert, sind nicht besonders eingeschränkt, vorteilhafterweise kann hierfür eine einfache Federvorrichtung verwendet werden, wie es in dem Beispiel beschrieben ist.

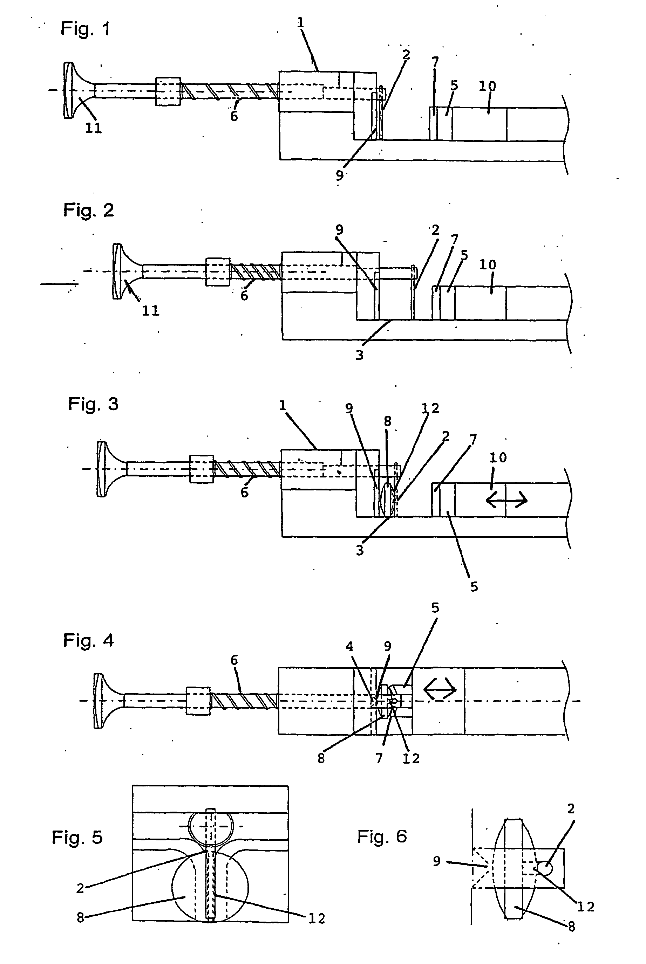

Die verwendete Bruchkante ist nicht besonders eingeschränkt, hierbei handelt es sich bevorzugt um eine Bruchkante, die stegartig ausgebildet ist, so daß ein Bruchsteg vorhanden ist, der eine Bruchfläche aufweist, wie es beispielsweise in Figur 6 dargestellt wird. Die Bruchkante kann aber auch beispielsweise als Schneide ausgestattet sein. Auch andere Ausgestaltungen sind denkbar. Wichtig ist, daß parallel zu dem Fixierstift auf der Seite der Tablette, die der Tablettenseite gegenüberliegt, die die Bruchkerbe aufweist, eine Vorrichtung vorhanden ist, die der Kraft, die an die Tablette angreift, eine entsprechende Gegenkraft entgegensetzt, so daß die Tablette kontrolliert zerbricht.

Die Kraft wird mit den Bruchbacken auf die Tablette übertragen, d.h. die Bruchbacken drücken die Tablette gegen die Bruchkante und zwar von derjenigen Seite aus, an der der Fixierstift an der Tablette anliegt.

Erfindungsgemäß bevorzugt erfolgt die Übertragung der Kraft durch die Bruchbacken auf die Tablette so, daß die Kraft möglichst flächig einwirkt, so daß das übliche Brechen der Tablette durch den Patienten, das in der Regel mit den Fingern erfolgen wird, möglichst gut simuliert wird. Dies kann beispielsweise durch geeignet ausgebildete Puffer, die beweglich an den Bruchbacken angebracht sind, bewirkt werden. Die Puffer sind bevorzugt elastisch ausgebildet, so daß sie sich gut der Form und der Wölbung der Tablette anpassen können.

Mit dem erfindungsgemäßen Verfahren können selbstverständlich auch Tabletten untersucht werden, die mehr als eine Bruchkerbe aufweisen. Insbesondere ist es möglich, daß gegenüber der ersten Bruchkerbe, an der der Fixierstift angreift, eine zweite Bruchkerbe vorhanden ist, die sich damit auf der Seite der Tablette befindet, auf der die Bruchkante angreift.

Da bei der Durchführung des erfindungsgemäßen Verfahrens die zu untersuchende Tablette mit einem Fixierstift entlang der Bruchkerbe stets parallel zur Bruchkante fixiert wird und die Kraft auf reproduzierbare Art und Weise stets an den gleichen Stellen der Tablette ansetzt, wird ein sehr genaues und in hohem Maße reproduzierbares Verfahren zur Bestimmung der Teilbarkeit und der Teilgenauigkeit von Tabletten zur Verfügung gestellt. Das Verfahren simuliert darüber hinaus sehr gut den in der Praxis wohl am häufigsten verwendeten Vorgang beim Brechen von Tabletten durch den Patienten, nämlich daß die Kraft mit den Daumen rechts und links der Bruchkerbe auf die Tablette angewendet wird. Das erfindungsgemäße Verfahren ist daher für die reproduzierbare und genaue Ermittlung der wesentlichen Parameter von teilbaren Tabletten ausgezeichnet geeignet.

Erfindungsgemäß wird ebenfalls eine Vorrichtung zur Verfügung gestellt, die sich zur Durchführung des erfindungsgemäßen Verfahrens eignet. Die erfindungsgemäße Vorrichtung kann selbstverständlich auch beispielsweise beim Arzt, in der Apotheke oder im Privathaushalt zur genauen und reproduzierbaren Teilung von teilbaren Tabletten verwendet werden. In der Praxis wird dies aber aus wirtschaftlichen Gründen wahrscheinlich eine eher untergeordnete Anwendung darstellen.

Die erfindungsgemäße Vorrichtung, die zur Durchführung des erfindungsgemäßen Verfahrens und damit zur Bestimmung der Teilbarkeit und der Teilgenauigkeit einer Tablette 8 mit zumindest einer Bruchkerbe 12 besonders geeignet ist, weist eine Vorschubeinrichtung 10, Bruchbacken 5 und eine Bruchkante 4 auf. Die Vorrichtung ist dadurch gekennzeichnet, daß sie einen Fixierstift 2 aufweist, der Bestandteil einer Einspannvorrichtung (1) ist und der so ausgelegt ist, daß er entlang der Bruchkerbe 12 der Tablette 8 angelegt werden kann und die Tablette 8 mit der Seite, die der Seite gegenüberliegt, die die Bruchkerbe 12 aufweist, gegen die Bruchkante 4 derart fixieren kann, daß der Fixierstift 2 und die Bruchkante 4 parallel zueinander liegen und die Vorschubeinrichtung (10) derart mit den Bruchbacken (5) verbunden ist, dass sie diese beidseitig des Fixierstifts (2) gegen die Tablette (8) drücken kann.

Der Fixierstift 2 ist Teil einer Einspannvorrichtung 1, die eine Vorrichtung umfaßt, die dafür sorgt, daß der Fixierstift 2 die Tablette 8 gegen die Bruchkante 4 fixiert. Bevorzugt ist diese Vorrichtung eine Feder 6, so daß der Fixierstift 2 die Tablette mit der Federkraft gegen die Bruchkante 4 preßt.

Bevorzugt weist die erfindungsgemäße Vorrichtung eine Auflagefläche 3 auf, auf der die Tabletten mit ihrer schmalen Seite aufliegen können.

Die Bruchbacken 5 sind bevorzugt an der Vorschubeinheit 10 befestigt und weisen bevorzugt Pufferelemente 7 auf, die bevorzugt elastisch und/oder beweglich angeordnet sind und so ausgestaltet sind, daß sie flächig eine Kraft auf die Tablette übertragen können. Die Bruchbacken 5 und die gegebenenfalls vorhandenen Puffer 7 sind bevorzugt derart angeordnet, daß sie zu beiden Seiten des Fixierstifts 2 jeweils eine gleichmäßige Kraft auf die Tablette 8 übertragen können. Die Vorschubeinrichtung 10 bewegt die Bruchbacken 5 gleichmäßig vorwärts (z.B. über einen Schrittmotor), so daß die Bruchbacken 8 bzw. die daran befestigten Puffer 7 gegen die Tablette drücken, bis diese zerbricht. Die bevorzugt feststehende Bruchkante 4 stellt die Gegenkraft zur Verfügung.

Die Bruchkante 4 ist bevorzugt ein Bruchsteg 4, der eine Bruchfläche 9 aufweist. Durch Verwendung eines Bruchstegs anstelle einer Schneide wird die Fixierung der Tablette durch den Fixierstift erleichtert. Wie vorstehend bei der Beschreibung des erfindungsgemäßen Verfahrens ausgeführt, sind aber auch andere Ausgestaltungen der Bruchkante möglich. Die Bruchfläche 9 weist bevorzugt eine Breite von 0,2 bis 2 mm, stärker bevorzugt von 0,5 bis 1,5 mm auf. Die Länge ist nicht besonders eingeschränkt und wird in der Regel derart gewählt, daß sie größer ist als der Durchmesser der zu teilenden Tabletten.

Bevorzugt weist die Vorschubeinheit 10 einen Dehnungsmeßstreifen auf, mit dem beim Bruchvorgang die Brechkraft der Tablette 8 bestimmt werden kann. Der Dehnungsmeßstreifen ist bevorzugt mit dem Schrittmotor der Vorschubeinheit 10 gekoppelt, so daß beim Brechen der Tablette und dem damit verbundenen Absinken der Druckkraft die Bruchbacken in Gegenrichtung, also von der Tablette 8 weg, bewegt werden. Konkrete Ausgestaltungen des Schrittmotors und des Dehnungsmeßstreifens sind dem Fachmann bekannt.

Dadurch, daß die am Bruchbacken 5 bevorzugt befestigten Puffer 7 den beim Brechvorgang erzeugten Druck flächig auf die Tablette 8 übertragen können, wird der Druck des menschlichen Fingers simuliert, wenn der Patient die Tablette ohne Hilfe einer dafür geeigneten Vorrichtung bricht.

Der in dem erfindungsgemäßen Verfahren verwendete bzw. in der erfindungsgemäßen Vorrichtung vorhandene Fixierstift muß so ausgestaltet sein, daß er die Tablette so ausrichten kann, daß die Bruchkerbe der Tablette parallel zu der Bruchkante liegt. Damit muß der Fixierstift so beschaffen sein, daß er in die Bruchkerbe eingreifen kann.

Bevorzugt ist der Fixierstift von zylinderförmiger Gestalt. Der Zylinder weist bevorzugt einen Durchmesser auf, der in der gleichen Größenordnung ist, wie die Breite der Bruchkerbe der Tablette. Der Durchmesser des Zylinders kann aber auch darunter oder darüber liegen, solange gewährleistet ist, daß der Fixierstift die Tablette anhand ihrer Bruchkerbe exakt ausrichten kann. In der Regel wird der Durchmesser des Zylinders im Bereich von 0,1 bis 3,0 mm, bevorzugt im Bereich von 0,2 bis 2,0 mm liegen, stärker bevorzugt im Bereich von 0,5 bis 1,5 mm.

Die Form des Fixierstifts kann auch von der Zylinderform abweichen. Z.B. kann die Grundfläche des Fixierstifts statt eines Kreises (wie bei einer Zylinderform) eine Ellipse, ein Rechteck oder ein Dreieck bilden, wobei dann für die Kantenlängen die vorstehend genannten Werte gelten. Bevorzugt ist allerdings die Zylinderform.

Da der Fixierstift nur zur Fixierung der Tablette dient, ist es nicht erforderlich, daß er großen Belastungen standhalten kann, wie sie beispielsweise auftreten würden, wenn die Kraft zur Teilung der Tablette über den Fixierstift auf die Tablette übertragen würde. Allerdings hat es sich als vorteilhaft herausgestellt, wenn der Stift möglichst starr ist, also keine Flexibilität aufweist. Entsprechend sollte auch die Befestigung an der Einspannvorrichtung derart sein, daß der Stift keine Beweglichkeit in Bezug auf die Einspannvorrichtung aufweist, sondern streng der von der Einspannvorrichtung vorgegebenen Bewegung folgt. Ein geeignetes Material für den Fixierstift ist z.B. Edelstahl, andere Materialien können aber ebenfalls verwendet werden.

Die vorliegende Erfindung ermöglicht es Tabletten mit vorgesehener Sollbruchstelle reproduzierbar zu teilen. Infolgedessen ist eine sichere Bestimmung der Teilbarkeit und der Teilgenauigkeit möglich.

Ein Ausführungsbeispiel der Erfindung wird anhand der Figuren 1 bis 6 erläutert, die eine besonders bevorzugte Ausgestaltung der erfindungsgemäßen Vorrichtung zeigen.

Die Figuren zeigen:
- Figur 1:: Seitenansicht einer bevorzugten erfindungsgemäßen Vorrichtung ohne Tablette (Ausgangsposition)
- Figur 2:: Seitenansicht einer bevorzugten erfindungsgemäßen Vorrichtung ohne Tablette (Einlegeposition)
- Figur 3:: Seitenansicht einer bevorzugten erfindungsgemäßen Vorrichtung mit fixierter, ungeteilter Tablette
- Figur 4:: Draufsicht einer bevorzugten erfindungsgemäßen Vorrichtung mit fixierter Tablette kurz vor dem Teilen
- Figur 5:: Vorderansicht der fixierten Tablette in einer bevorzugten erfindungsgemäßen Vorrichtung
- Figur 6:: Draufsicht der fixierten Tablette in einer bevorzugten erfindungsgemäßen Vorrichtung.

In Figur 1 ist die Einspannvorrichtung 1 in leerem Zustand, d.h. ohne Tablette, dargestellt. Die Vorschubeinheit 10 befindet sich in Ausgangsstellung, die Feder 6 ist ohne Spannung. Der Fixierstift 2 liegt an der Bruchfläche 9 an.

Zum Einlegen einer Tablette wird der Griff 11 in Richtung Vorschubeinheit 10 gedrückt, wodurch die Feder 6 zusammengedrückt wird und der Fixierstift 2 sich in Richtung Vorschubeinheit 10 bewegt.

In dieser Position, wie in Figur 2 dargestellt, wird nun die zu prüfende Tablette von Hand oder mit Hilfe einer Pinzette so zwischen Bruchfläche 9 und Fixierstift 2 eingelegt, daß die Tablette mit der Bruchkerbe 12 in Richtung Fixierstift 2 zeigt und auf der Auflagefläche 3 aufliegt. Die Bruchkerbe 12 muß zudem parallel zum Fixierstift 2 ausgerichtet sein. Löst man nun den Druck am Griff 11, so bewegt sich der Fixierstift 2 in Richtung Tablette, positioniert diese durch senkrechte Ausrichtung der Bruchkerbe und drückt die Tablette durch die Kraft der Feder 6 leicht gegen die Bruchfläche 9 des Bruchstegs 4.

In der Figur 5 (Vorderansicht) und 6 (Draufsicht) ist im Detail dargestellt, wie die Tablette 8 durch den Fixierstift 2 an der Bruchkerbe 12 ausgerichtet und gegen die Bruchfläche 9 gedrückt wird.

In Figur 3 ist die Einspannvorrichtung 1 mit fixierter Tablette 8 dargestellt. In dieser Position wird nun der Antrieb der Vorschubeinheit 10 gestartet, welche die Bruchbacken 5 mit konstanter Geschwindigkeit und Kraft gegen die Tablette 8 drückt.

Figur 4 zeigt die Position, in der die an den Bruchbacken 5 angebrachten Puffer 7 gegen die Tablette 8 drücken. Die Puffer 7 passen sich aufgrund ihrer elastischen Beschaffenheit der Form und Wölbung der Tablettenoberfläche an. Bei einer bestimmten Kraft, der sogenannten Brechkraft, kann die Tablette dem aufgebrachten Druck nicht mehr widerstehen und zerbricht entlang der Bruchkerbe. Dabei fällt die von der Tablette erzeugte Gegenkraft ab, wodurch die Vorschubeinheit stoppt und sich zurück in die Ausgangsposition bewegt (Figur 1).

Im Moment des Brechens der Tablette wird die von der Vorschubeinheit aufgebrachte Kraft gemessen, welche der Brechkraft entspricht. Die geteilte Tablette bleibt auf der Auflagefläche liegen von wo sie von Hand oder mit einer Pinzette aufgenommen werden kann. Die einzelnen Tablettenteile können dann durch bekannte Methoden auf Gehalts- und Massegleichförmigkeit untersucht werden, und damit werden Angaben über die Teilgenauigkeit auf reproduzierbare Art und Weise erhalten. Der Fixierstift bewegt sich nach dem Brechen der Tablette durch die Kraft der Feder 6 wieder in die Ausgangsposition zurück (Figur 1). Falls erforderlich kann die Auflagefläche 3 mit einem Pinsel von Tablettenrückständen befreit werden.

Das Gerät ist nun zum Teilen einer weiteren Tablette bereit.

## Patentansprüche

1. Verfahren zur Bestimmung der Teilbarkeit und der Teilgenauigkeit einer Tablette, bei dem eine Tablette, die auf zumindest einer Seite zumindest eine Bruchkerbe aufweist, entlang dieser Bruchkerbe gebrochen wird, **dadurch gekennzeichnet, daß** an der Seite der Tablette, die diese Bruchkerbe aufweist, ein Fixierstift entlang der Bruchkerbe angelegt wird, so daß die Tablette mit der Seite, die der Seite gegenüberliegt, die diese Bruchkerbe aufweist, gegen eine Bruchkante derart fixiert wird, daß die Bruchkante und der Fixierstift parallel zueinander liegen und dann über Bruchbacken, die beidseitig des Fixierstifts auf die Tablette einwirken, solange Kraft auf die Tablette ausgeübt wird, bis die Tablette zerbricht.

2. Verfahren nach Anspruch 1, **dadurch gekennzeichnet, daß** die Bruchkante ein Bruchsteg mit einer Bruchfläche ist.

3. Verfahren nach Anspruch 1 oder 2, **dadurch gekennzeichnet, daß** die Bruchbacken von einer Vorschubeinheit auf die Tablette zu bewegt werden.

4. Verfahren nach Anspruche 3, **dadurch gekennzeichnet, daß** die Vorschubeinheit mit einem Dehnungsmeßstreifen ausgestattet ist, mit dem beim Bruchvorgang die Brechkraft der Tablette bestimmt werden kann.

5. Verfahren nach Anspruch 4, **dadurch gekennzeichnet, daß** die Vorschubeinheit einen Schrittmotor aufweist und der Dehnungsmeßstreifen mit dem Schrittmotor derart gekoppelt ist, dass beim Brechen der Tablette und dem damit verbundenen Absinken der Druckkraft die Druckbacken in Gegenrichtung von der Tablette wegbewegt werden.

6. Vorrichtung zur Bestimmung der Teilbarkeit und der Teilgenauigkeit einer eine Bruchkerbe (12) aufweisenden Tablette (8), mit einer Vorschubeinrichtung (10), Bruchbacken (5) und einer Bruchkante (4), **dadurch gekennzeichnet, dass** sie einen Fixierstift (2) aufweist, der Bestandteil einer Einspannvorrichtung (1) ist und der so ausgelegt ist, dass er entlang der Bruchkerbe (12) der Tablette (8) angelegt werden kann und die Tablette (8) mit der Seite, die der Seite gegenüberliegt, die die Bruchkerbe (12) aufweist, gegen die Bruchkante (4) derart fixieren kann, dass der Fixierstift (2) und die Bruchkante (4) parallel zueinander liegen und die Vorschubeinrichtung (10) derart mit den Bruchbacken (5) verbunden ist, dass sie diese beidseitig des Fixierstifts (2) gegen die Tablette (8) drücken kann.

7. Vorrichtung nach Anspruch 6, **dadurch gekennzeichnet, daß** die Einspannvorrichtung (1) eine Feder (6) zur Fixierung der Tablette (8) mit dem Fixierstift (2) aufweist.

8. Vorrichtung nach Anspruch 6 oder 7, **dadurch gekennzeichnet, daß** die Bruchbacken (5) Pufferelemente (7) aufweisen.

9. Vorrichtung nach Anspruch 8, **dadurch gekennzeichnet, daß** die Pufferelemente (7) derart ausgestaltet sind, daß sie den Druck flächig zu beiden Seiten des Fixierstifts (2) auf die Tablette 8 übertragen können.

10. Vorrichtung nach einem der Ansprüche 6 bis 9, **dadurch gekennzeichnet, daß** die Bruchkante (4) ein Bruchsteg (4) ist, der eine Bruchfläche (9) aufweist.

11. Vorrichtung nach einem der Ansprüche 6 bis 10, **dadurch gekennzeichnet, daß** die Vorschubeinheit (10) einen Schrittmotor umfaßt.

12. Vorrichtung nach Anspruch 11, **dadurch gekennzeichnet, daß** die Vorschubeinheit (10) einen Dehnungsmeßstreifen aufweist, mit dem beim Bruchvorgang die Brechkraft der Tablette (8) bestimmt werden kann und der mit dem Schrittmotor gekoppelt ist.

## Claims

1. Method for determining the breakability and the breaking accuracy of a tablet, in which a tablet which has at least one breaking notch on at least one side is split along this breaking notch, **characterised in that** a fixing pin is placed against the side of the tablet having said breaking notch, along the breaking notch, so that the tablet is so fixed against a breaking edge by the side opposite the side having the breaking notch that the breaking edge and the fixing pin are disposed parallel to one another and force is then exerted on the tablet via breaking jaws acting on the tablet on both sides of the fixing pin until the tablet fractures.

2. Method according to claim 1, **characterised in that** the breaking edge is a breaking bar having a breaking face.

3. Method according to claim 1 or 2, **characterised in that** the breaking jaws are moved towards the tablet by a feed unit.

4. Method according to claim 3, **characterised in that** the feed unit is equipped with a strain gauge with which the breaking force of the tablet can be determined during the breaking process.

5. Method according to claim 4, **characterised in that** the feed unit includes a stepper motor and the strain gauge is so coupled to the stepper motor that, upon the fracturing of the tablet and the associated drop in the pressure force, the pressure jaws are moved in the opposite direction, away from the tablet.

6. Device for determining the breakability and the breaking accuracy of a tablet (8) having a breaking notch (12), comprising a feed device (10), breaking jaws (5) and a breaking edge (4), **characterised in that** it includes a fixing pin (2) which is a component of a clamping device (1) and is so designed that it can be placed along the breaking notch (12) of the tablet (8) and can so fix the tablet (8) against the breaking edge (4) by the side disposed opposite the side having the breaking notch (12) that the fixing pin (2) and the breaking edge (4) are disposed parallel to one another, and the feed device (10) is so connected to the breaking jaws (5) that it can press them against the tablet (8) on both sides of the fixing pin (2).

7. Device according to claim 6, **characterised in that** the clamping device (1) includes a spring (6) for fixing the tablet (8) with the fixing pin (2).

8. Device according to claim 6 or 7, **characterised in that** the breaking jaws (5) include buffer elements (7).

9. Device according to claim 8, **characterised in that** the buffer elements (7) are so configured that they can transmit the pressure to the tablet (8) in a planar manner on both sides of the fixing pin (2).

10. Device according to any one of claims 6 to 9, **characterised in that** the breaking edge (4) is a breaking bar (4) which has a breaking face (9).

11. Device according to any one of claims 6 to 10, **characterised in that** the feed unit (10) includes a stepper motor.

12. Device according to claim 11, **characterised in that** the feed unit (10) includes a strain gauge with which the breaking force of the tablet (8) can be determined during the breaking process and which is coupled to the stepper motor.

## Revendications

1. Procédé de détermination de la divisibilité et de la précision de division d'un comprimé, dans lequel un comprimé présentant, sur au moins un côté, au moins une entaille de rupture, est rompu le long de cette entaille de rupture, **caractérisé en ce que**, sur le côté du comprimé présentant cette entaille de rupture, une tige de maintien est appliquée le long de l'entaille de rupture, de sorte que le comprimé est maintenu par le côté opposé au côté présentant cette entaille de rupture contre une arête de rupture de telle sorte que cette arête de rupture et la tige de maintien sont disposées parallèlement l'une par rapport à l'autre et qu'ensuite, par l'intermédiaire de mâchoires de rupture agissant sur le comprimé de part et d'autre de la tige de maintien, une force est exercée sur le comprimé jusqu'à ce que le comprimé se rompe.

2. Procédé selon la revendication 1, **caractérisé en ce que** l'arête de rupture est une barrette de rupture présentant une surface de rupture.

3. Procédé selon la revendication 1 ou 2, **caractérisé en ce que** les mâchoires de rupture sont déplacées jusque sur le comprimé par le moyen d'un dispositif d'avance.

4. Procédé selon la revendication 3, **caractérisé en ce que** le dispositif d'avance est équipé d'une jauge de contrainte par le moyen de laquelle la force de rupture du comprimé peut être déterminée pendant le processus de rupture.

5. Procédé selon la revendication 4, **caractérisé en ce que** le dispositif d'avance présente un moteur pas à pas et que la jauge de contrainte est couplée avec le moteur pas à pas de telle manière que, lors de la rupture du comprimé et de la baisse correspondante de la force de pression, les mâchoires de rupture peuvent être éloignées du comprimé en direction opposée.

6. Dispositif de détermination de la divisibilité et de la précision de division d'un comprimé (8) présentant une entaille de rupture (12), en utilisant un dispositif d'avancement (10), des mâchoires de rupture (5) et une arête de rupture (4), **caractérisé en ce qu'**il présente une tige de maintien (2), laquelle fait partie d'un dispositif de serrage (1), et qui est disposée de telle sorte qu'elle est appliquée le long de l'entaille de rupture (12) du comprimé (8), et qui peut maintenir le comprimé (8) par le côté opposé au côté présentant cette entaille de rupture (12) contre l'arête de rupture (4) de telle manière que la tige de maintien (2) et l'arête de rupture (4) sont disposées parallèlement l'une par rapport à l'autre, et que le dispositif d'avancement (10) est relié aux mâchoires de rupture (5) de telle sorte que celles-ci peuvent appuyer sur le comprimé (8) de part et d'autre de la tige de maintien (2).

7. Dispositif selon la revendication 6, **caractérisé en ce que** le dispositif de serrage (1) présente un ressort (6) pour maintenir le comprimé (8) par le moyen de la tige de maintien (2).

8. Dispositif selon la revendication 6 ou 7, **caractérisé en ce que** les mâchoires de rupture (5) présentent des éléments formant amortisseurs (7).

9. Dispositif selon la revendication 8, **caractérisé en ce que** les éléments formant amortisseurs (7) sont conçus de telle manière qu'ils peuvent transmettre la pression en surface de part et d'autre de la tige de maintien sur le comprimé (8).

10. Dispositif selon l'une quelconque des revendications 6 à 9, **caractérisé en ce que** l'arête de rupture (4) est une barrette de rupture (4) présentant une surface de rupture (9).

11. Dispositif selon l'une quelconque des revendications 6 à 10, **caractérisé en ce que** le dispositif d'avance (10) comporte un moteur pas à pas.

12. Dispositif selon la revendication 11, **caractérisé en ce que** le dispositif d'avance (10) présente une jauge de contrainte, par le moyen de laquelle la force de rupture du comprimé (8) peut être déterminée pendant le processus de rupture, et qui est couplée avec le moteur pas à pas.
